# EUROPÄISCHE PATENTANMELDUNG

(11) **EP 0 865 016 A1**
(43) Veröffentlichungstag der Anmeldung: **16.09.1998**
(21) Anmeldenummer: 98104540.4
(22) Anmeldetag: 13.03.1998
(51) Int. Cl.: G09F 7/12, A61L 9/12, G09F 7/18

(54) **Informationsträger mit Duftspender**

(30) Priorität: 13.03.1997 DE 29704563 U
(71) Anmelder: Fritzsche, Albrecht, 74420 Gaildorf (DE)
(72) Erfinder: Fritzsche, Albrecht, 74420 Gaildorf (DE)
(74) Vertreter: Dreiss, Fuhlendorf, Steimle & Becker, Patentanwälte

(57) **Zusammenfassung**

Es ist eine Vorrichtung zur Befestigung von Gegenständen beschrieben, insbesondere ein Informationsträger (1) oder dergleichen. Es ist eine Halterung, insbesondere ein Saugnapf (2) vorgesehen, an der ein flexibler, länglicher Träger (3) gehalten ist, an dem der Gegenstand (4) anbringbar ist. Als Gegenstand (4) bzw. in dem Gegenstand (4) ist ein Duftspender (10) vorgesehen.

## Beschreibung

Die Erfindung betrifft eine Vorrichtung zur Befestigung von Gegenständen, insbesondere einen Informationsträger oder dergleichen, mit einer Halterung, insbesondere mit einem Saugnapf, an der ein flexibler, länglicher Träger gehalten ist, an dem ein Gegenstand anbringbar ist.

Eine derartige Vorrichtung ist aus dem deutschen Gebrauchsmuster G 90 16 658 U1 bekannt. Dort kann die Vorrichtung mit einem Saugnapf an einer ebenen Fläche befestigt werden. An dem Saugnapf ist eine Bandfeder angebracht, die parallel zu der Fläche angeordnet ist. Die Bandfeder ist derart ausgestaltet, dass sie nur in einer zu der Fläche parallelen Ebene schwenkbar ist, jedoch nicht in einer senkrecht zu der Flache ausgerichteten Ebene. Am freien Ende der Bandfeder kann ein Gegenstand befestigt sein, der mit einer Information versehen ist. Insbesondere ist der Gegenstand dazu geeignet, blattförmige oder figürliche Darstellungen, beispielsweise Werbemittel, Hinweisschilder, Preislisten, Speise- und Getränkekarten, Drucksachen oder dergleichen darzustellen.

Aufgabe der Erfindung ist es, den Informationsgehalt der bekannten Vorrichtung weiter zu erhöhen.

Diese Aufgabe wird bei einer Vorrichtung der eingangs genannten Art erfindungsgemäß dadurch gelöst, dass als Gegenstand bzw. in dem Gegenstand ein Duftspender vorgesehen ist.

Während die bekannte Vorrichtung nur zur Darstellung von optischen Informationen geeignet ist, ist es bei der erfindungsgemäßen Vorrichtung möglich, "Duftinformationen" auszusenden, also einen in der Umgebung der Vorrichtung sich befindenden Menschen über den Geruchssinn anzusprechen. Auf diese Weise wird eine ganz neue Art der Informationsverbreitung eröffnet.

Dabei ist es besonders vorteilhaft, dass aufgrund des flexiblen Trägers der Duftspender leicht in eine Bewegung zu versetzen ist. Durch diese Bewegung wird der Duft aus dem Duftspender gleichmäßig an die Umgebung abgegeben und verteilt. Es sind also keine besonderen Maßnahmen erforderlich, um den Duft an die Umgebung zu verteilen, sondern dies geschieht praktisch automatisch. Insbesondere wird die Bewegung dabei durch Luftbewegungen erzeugt, die mehr oder weniger überall vorhanden sind.

Des Weiteren ist es vorteilhaft, dass der Duftspender nicht nur für sich, also als einzige Information vorgesehen sein kann, sondern es ist ebenfalls möglich, den Duftspender mit den bekannten optischen Informationen zu kombinieren. Dies hat den Vorteil, dass der Duftspender innerhalb der optischen Informationen untergebracht werden kann, und damit als solcher nicht mehr sichtbar ist. Des Weiteren kann dadurch erreicht werden, dass die von dem Duftspender abgegebenen Informationen durch die optische Informationen verstärkt werden können.

Bei einer vorteilhaften Weiterbildung der Erfindung ist der Duftspender mit einem optischen Hinweis auf den Ursprung des Dufts versehen. Dabei ist es besonders zweckmäßig, wenn der Duftspender mit der Firma oder dem Logo des den Duft herstellenden Unternehmens versehen ist. Als optische Informationen können also die Firma oder das Logo beispielsweise eines Parfumherstellers vorgesehen sein. Des Weiteren ist es möglich, auch noch den Namen eines Parfums auf dem Informationsträger anzubringen. Diese optischen Informationen können dann mit dem speziellen Parfum kombiniert werden, das als Duftspender auf den Informationsträger aufgebracht wird. Die Werbewirksamkeit wird auf diese Weise wesentlich erhöht. Ein "Betrachter" sieht nicht nur den Namen des Parfums und den Namen der herstellenden Firma, sondern er kann das Parfum auch sofort mit seinem Geruchssinn "wahrnehmen" und feststellen.

Bei einer weiteren vorteilhaften Weiterbildung der Erfindung weist der Duftspender eine figürliche Form auf, beispielsweise die Gestalt einer Pflanze oder eines Tieres oder eine bildliche Darstellung, beispielsweise das Foto eines Menschen. Handelt es sich bei dem Namen eines Parfums beispielsweise um den Namen berühmter Sportler/innen oder Künstler/innen oder dergleichen, so kann der Duftspender die figürliche Form und/oder die bildliche Darstellung dieser berühmten Person aufweisen. Die Werbewirksamkeit des Informationsträgers wird dadurch weiter erhöht, da nunmehr nicht nur der Name des Parfums, sondern auch dessen figürliche oder bildliche Darstellung von einem Betrachter sofort erkannt werden kann.

Bei einer weiteren vorteilhaften Weiterbildung der Erfindung weist der Duftspender die figürliche Form von Hinweis- oder Warnzeichen oder die bildliche Darstellung von Hinweis- oder Warnzeichen auf. Auf diese Weise kann insbesondere ein unangenehmer Geruch mit einem auf eine Gefahr hinweisenden Warnzeichen verbunden werden. Ein "Betrachter" dieses Informationsträgers wird damit nicht nur durch das optische Warnzeichen auf die Gefahr hingewiesen, sondern darüber hinaus auch durch den unangenehmen Geruch.

Besonders zweckmäßig ist es, wenn der Duftspender austauschbar ist. Damit kann mit ein- und derselben Vorrichtung durch Anbringung verschiedener Duftspender eine unterschiedliche Werbewirkung erzielt werden. Des Weiteren ist es möglich, einen Duftspender, dessen Duft verbraucht ist, durch einen neuen Duftspender zu ersetzen.

Bei einer weiteren vorteilhaften Weiterbildung der Erfindung ist der Träger in alle Richtungen schwenkbar bzw. um seine Längsachse drehbar. Auf diese Weise wird erreicht, dass der Duftträger besonders leicht in eine Schwenkbewegung oder in eine Drehbewegung versetzt wird, ohne das hierzu besondere weitere Maßnahmen erforderlich wären. Durch die Schwenk- oder Drehbewegung wird dann der Duft gleichmäßig an die Umgebung abgegeben und verteilt.

Besonders zweckmäßig ist es, wenn der Träger etwa parallel zu einer ebenen Fläche angeordnet ist, wobei der Abstand so groß ist, dass der Duftspender bei normalen Schwenkbewegungen die Fläche nicht berührt. Der Duftspender ist damit nicht nur parallel zu der Fläche schwenkbar, sondern in alle Richtungen. Damit wird einerseits die Werbewirksamkeit erhöht, und andererseits wird die Abgabe des Dufts an die Umgebung erleichtert.

Ebenfalls ist es möglich, dass der Träger etwa senkrecht zu einer ebenen Fläche angeordnet ist. Dies hat den wesentlichen Vorteil, dass der Duftspender unter keinen Umständen mit der Fläche in Berührung kommen kann.

Bei einer vorteilhaften Weiterbildung der Erfindung ist der Träger aus der etwa parallelen Stellung in die etwa senkrechte Stellung verstellbar. Damit ist es möglich, mit ein- und derselben Vorrichtung den Duftspender etwa parallel oder etwa senkrecht zu einer ebenen Fläche anzuordnen.

Weitere Merkmale, Anwendungsmöglichkeiten und Vorteile der Erfindung ergeben sich aus der nachfolgenden Beschreibung von Ausführungsbeispielen der Erfindung, die in der Zeichnung dargestellt sind. Dabei bilden alle beschriebenen oder dargestellten Merkmale für sich oder in beliebiger Kombination den Gegenstand der Erfindung, unabhängig von ihrer Zusammenfassung in den Patentansprüchen oder deren Rückbeziehung sowie unabhängig von ihrer Formulierung bzw. Darstellung in der Beschreibung bzw. in der Zeichnung.
- Figur 1: zeigt eine schematische Draufsicht auf einen erfindungsgemäßen Informationsträger;
- Figur 2: zeigt eine schematische Seitenansicht des Informationsträger der Figur 1 bei einer Anbringung an eine vertikale ebene Fläche; und
- Figur 3: zeigt eine schematische Seitenansicht des Informationsträgers der Figur 1 bei einer Anbringung an eine etwa horizontale ebene Fläche.

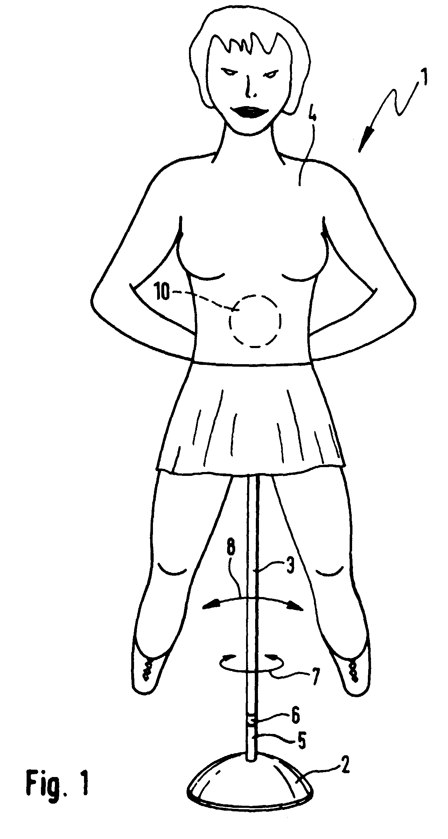

In den Figuren 1 bis 3 ist ein Informationsträger 1 dargestellt, der einen Saugnapf 2, einen Träger 3 sowie einen Gegenstand 4 aufweist. Der Träger 3 ist länglich ausgestaltet, und ist mit seinem einen Ende mit dem Saugnapf 2 und mit seinem anderen Ende mit dem Gegenstand 4 verbunden.

Der Saugnapf 2 ist derart ausgebildet, dass er an einer ebenen Fläche fest angebracht werden kann. Es ist ebenfalls möglich, anstelle des Saugnapfes 2 eine andere Halterung vorzusehen, mit der der Informationsträger an der ebenen Fläche befestigt werden kann. Beispielsweise kann es sich bei einer derartigen Halterung um eine Klebeplatte oder dergleichen handeln.

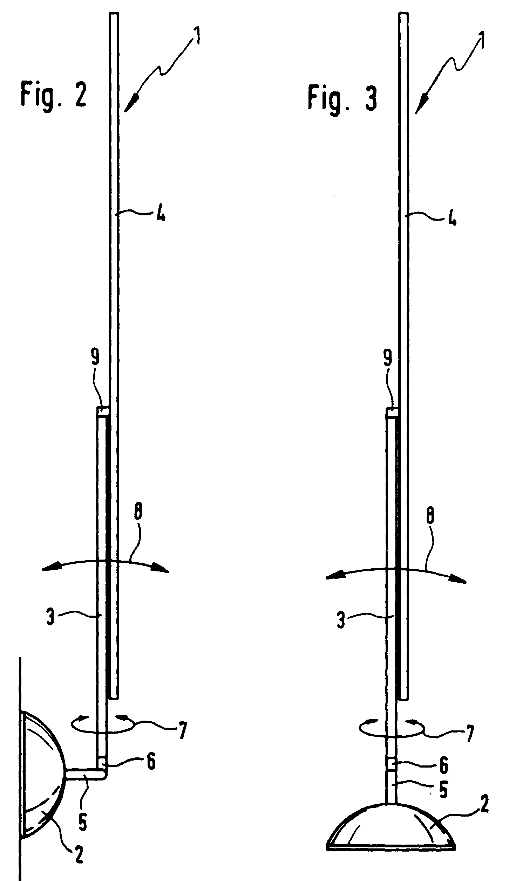

Der Saugnapf 2 ist über ein Verbindungsstück 5 mit dem Träger 3 verbunden. Das Verbindungsstück 5 ist, wie insbesondere den Figuren 2 und 3 zu entnehmen ist, derart an den Saugnapf 2 angebracht, dass es immer etwa senkrecht zu der ebenen Fläche angeordnet ist, an der der Saugnapf 2 befestigt ist.

Zwischen dem Verbindungsstück 5 und dem Träger 3 ist ein Element 6 vorgesehen, mit dem der Träger 3 einerseits aus einer Position senkrecht zum Verbindungsstück 5 in eine Position parallel zum Verbindungsstück 5 verschwenkt werden kann, und die andererseits dazu geeignet ist, dass der Träger 5 eine Drehbewegung 7 um seine Längsachse ausführen kann.

Die Schwenkbewegung des Trägers 3 im Hinblick auf das Verbindungsstück 5 ist insbesondere den Figuren 2 und 3 zu entnehmen, während die Drehbewegung 7 des Trägers 3 in allen Figuren 1 bis 3 angedeutet ist.

Der Träger 3 ist in der Form einer länglichen Stange ausgebildet. Der Träger 3 ist flexibel ausgestaltet. Beispielsweise kann es sich bei dem Träger 3 um einen Federstahldraht oder dergleichen handeln.

Damit ist es möglich, dass bei feststehendem Saugnapf 2 und damit feststehendem Verbindungsstück 5 der Träger 3 mit dem daran gehaltenen Gegenstand 4 eine Schwenkbewegung 8 ausführt. Diese Schwenkbewegung 8 hat keine Vorzugsrichtung, so dass der Gegenstand 4 in alle Richtungen schwenkbar ist.

An seinem Ende ist an dem Träger 3 der Gegenstand 4 befestigt. Zu diesem Zweck ist ein Element 9 vorgesehen, bei dem es sich beispielsweise um ein Clipselement oder dergleichen handeln kann. Dadurch ist es möglich, dass der Gegenstand 4 von dem Träger 3 gelöst und wieder angebracht werden kann. Der Gegenstand 4 kann dadurch gegen einen anderen Gegenstand ausgetauscht werden.

Erfindungsgemäß ist vorgesehen, dass innerhalb des Gegenstand 4 ein Duftspender 10 vorgesehen ist, oder dass der gesamte Gegenstand 4 als Duftspender 10 ausgebildet ist. Im ersten Fall kann dies beispielsweise dadurch erreicht werden, dass an einer bestimmten Stelle des Gegenstands 4 eine Dufttablette oder dergleichen vorgesehen ist. Diese kann insbesondere innerhalb des Gegenstands 4 versteckt sein oder einen figürlichen Bestandteil des Gegenstands darstellen. Im zweiten Fall ist es möglich, dass der gesamte Gegenstand 4 beispielsweise durch Einsprühen oder Tränken mit dem Duft versehen ist.

Wie mit Hilfe der Figur 1 angedeutet werden soll, kann der gesamte Gegenstand 4 oder - im zweiten Fall - der Duftspender 10 beispielsweise die figürliche Darstellung eines Menschen sein. Bei diesem Menschen kann es sich insbesondere um eine berühmte Persönlichkeit oder dergleichen handeln. Des Weiteren kann zusätzlich in irgendeiner Weise auf dem Gegenstand 4 bzw. auf dem Duftspender 10 auf den Namen des Menschen hingewiesen werden. Dieser Name stellt dann gleichzeitig den Namen eines Parfums dar. Bei dem Duft, den der Duftspender 10 ausstrahlt, handelt es sich dann genau um diesen Duft.

Des Weiteren ist es möglich, weitere und/oder andere Hinweise auf den Ursprung des Dufts auf dem Gegenstand 4 oder auf dem Duftspender 10 anzubringen, so beispielsweise die Firma des den Duft herstellenden Unternehmens oder deren Logo. Ebenfalls ist es möglich, dass, insbesondere wenn der Duft den Namen einer Pflanze oder eines Tieres trägt, der Gegenstand 4 oder - im zweiten Fall - der gesamte Duftspender 10 die figürliche Form und/oder die figürliche Darstellung oder das Foto oder dergleichen dieser Pflanze oder dieses Tieres ist.

In ähnlicher Weise ist es möglich, den Gegenstand 4 bzw. den Duftspender 10 in der Form oder mit der Darstellung eines Hinweis- oder Warnzeichens auszugestalten. In diesem Fall kann es sich bei dem Duft jedoch eher um einen unangenehmen Geruch handeln, mit dem beispielsweise auf eine Gefahr hingewiesen wird.

## Patentansprüche

1. Vorrichtung zur Befestigung von Gegenständen, insbesondere Informationsträger (1) oder dergleichen, mit einer Halterung, insbesondere mit einem Saugnapf (2), an der ein flexibler, länglicher Träger (3) gehalten ist, an dem ein Gegenstand (4) anbringbar ist, **dadurch gekennzeichnet**, daß als Gegenstand (4) bzw. in dem Gegenstand (4) ein Duftspender (10) vorgesehen ist.

2. Vorrichtung nach einem der vorstehenden Ansprüche, dadurch gekennzeichnet, daß der Duftspender (10) mit einem optischen Hinweis auf den Ursprung des Dufts versehen ist.

3. Vorrichtung nach Anspruch 3, dadurch gekennzeichnet, daß der Duftspender (10) mit der Firma oder dem Logo des den Duft herstellenden Unternehmens versehen ist.

4. Vorrichtung nach einem der vorstehenden Ansprüche, dadurch gekennzeichnet, daß der Duftspender (10) eine figürliche Form aufweist, beispielsweise die Gestalt einer Pflanze oder eines Tieres.

5. Vorrichtung nach einem der vorstehenden Ansprüche, dadurch gekennzeichet, daß der Duftspender (10) eine bildliche Darstellung aufweist, beispielsweise das Foto eines Menschen.

6. Vorrichtung nach einem der vorstehenden Ansprüche, dadurch gekennzeichnet, daß der Duftspender (10) die figürliche Form von Hinweis- oder Warnzeichen oder die bildliche Darstellung von Hinweis- oder Warnzeichen aufweist.

7. Vorrichtung nach Anspruch 1, dadurch gekennzeichnet, daß der Duftspender (10) austauschbar ist (9).

8. Vorrichtung nach einem der vorstehenden Ansprüche, dadurch gekennzeichnet, daß der Träger (3) in alle Richtungen schwenkbar ist (8).

9. Vorrichtung nach einem der vorstehenden Ansprüche, dadurch gekennzeichnet, daß der Träger (3) um seine Längsachse drehbar ist (6, 7).

10. Vorrichtung nach Anspruch 8 oder Anspruch 9, dadurch gekennzeichnet, daß der Träger (3) etwa parallel zu einer ebenen Fläche angeordnet ist, wobei der Abstand so groß ist, daß der Duftspender bei normalen Schwenkbewegungen die Fläche nicht berührt.

11. Vorrichtung nach einem der vorstehenden Ansprüche, dadurch gekennzeichnet, daß der Träger (3) etwa senkrecht zu einer ebenen Fläche angeordnet ist.

12. Vorrichtung nach Anspruch 10 und Anspruch 11, dadurch gekennzeichnet, daß der Träger (3) aus der etwa parallelen Stellung in die etwa senkrechte Stellung verstellbar ist (6).
